# EUROPEAN PATENT APPLICATION

(11) **EP 3 868 363 A1**
(43) Date of publication of application: **25.08.2021**
(21) Application number: 18936998.6
(22) Date of filing: 17.10.2018
(51) Int. Cl.: A61K 9/08, A61K 9/14, A61K 31/7048, A61K 47/40, A61P 35/00

(54) **TENIPOSIDE INJECTION SOLUTION HAVING GOOD DILUTION STABILITY AND PREPARATION METHOD THEREFOR**

(71) Applicant: Jiangsu Linghang Biological Technology Co., Ltd., Nanjing, Jiangsu 210038 (CN)
(72) Inventor: WANG, Gang, Nanjing, Jiangsu 210038 (CN); DONG, Xiangyu, Nanjing, Jiangsu 210038 (CN); ZHAO, Yanbo, Nanjing, Jiangsu 210038 (CN)
(74) Representative: Cabinet Beau de Loménie
(86) International application number: PCT/CN2018/110655
(87) International publication number: WO 2020/077564

(57) **Abstract**

Disclosed by the present invention are a teniposide injection solution having good dilution stability and a preparation method therefor. A teniposide injection solution or powder, comprising teniposide, cyclodextrin, stabilizer and additive; the mass ratio of teniposide: cyclodextrin is 1:5-20. Compared with a commercially available teniposide injection (VUMON), the teniposide preparation of the present invention does not contain polyoxyethylene castor oil, benzoic acid and N, N-dimethylacetamide, has fewer harmful side effects, reduces adverse reactions in patients, and facilitates good patient compliance. The teniposide preparation of the present invention has excellent dilution stability, no dilution or precipitation occurs during a diluting process, and the preparation may be stably stored for 12 hours without precipitation when diluted to the final concentration; meanwhile the preparation process is simple and costs are low.

## Description

### Technical field

The invention belongs to the technical field of pharmaceutical preparations, and specifically relates to a teniposide injection with good dilution stability and preparation method thereof.

### Background art

Teniposide, also known as Vumon or VM-26, is a semisynthetic derivative of podophyllotoxin, belonging to plant-derived anti-tumor drugs. It is a cycle-specific cytotoxic drug that inhibits DNA topoisomerase II, causing double-stranded or single-stranded damage to stop cell mitosis at the late S or early G 2 stage, thereby hindering tumor cell division and inhibiting tumor growth. Teniposide has a significant curative effect, and its biological activity is 5-10 times that of the same type of the drug etoposide, and clinical data show that teniposide has few side effects and low toxicity, and has no significant effect on liver and kidney functions. Because of its broad-spectrum anti-tumor activity, teniposide usually clinically combined with other anti-cancer drugs is mainly used for malignant lymphoma, Hodgkin's disease, acute lymphoblastic leukemia, glioblastoma, Empty hemangioma, astrocytoma, bladder cancer, neuroblastoma and other solid tumors in children. It is also used in the treatment of small cell lung cancer, ovarian cancer, breast cancer, multiple myeloma, non-small cell lung cancer, etc.; moreover, due to its neutral lipophilic properties, it can pass through the blood-brain barrier and has become one of the preferred chemotherapy drugs for clinical treatment of brain tumors. Teniposide injection was formally approved by the US FDA in 1992 for the chemotherapy of pediatric acute lymphoblastic leukemia, with the trade name VUMON. China succeeded in preparing its generic drug at the beginning of the 21st century, with a product name Banglai.

Since teniposide itself is almost insoluble in water, it must be dissolved in an organic solvent, and a surfactant must be added to help solubilize it. The prescription composition of the commercially available preparation is: each ampoule (5ml) contains 50mg teniposide, 150mg benzyl alcohol, 300mg N,N-dimethylacetamide, 2.5g Cremophor EL , 42.7% (v/v) absolute ethanol. For clinical use alone, it is administered at a body surface area of 50-100 mg/m², diluted with 5% glucose solution or saline and then instilled intravenously. The main problem in the clinical use of commercially available preparations is that a large amount of polyoxyethylene castor oil in the prescription can cause the release of histamine in the body after use and cause severe allergic reactions including bronchospasm, shortness of breath, fatigue, low blood pressure, etc. For this reason, doctors must understand the patient's allergy history, such as whether they are allergic to teniposide or castor oil, etc.; during clinical use, medical staff must closely observe the patient's conditions and use antihistamines if necessary to relieve the severe allergic reaction caused by polyoxyethylene castor oil, resulting in very inconvenient clinical use, and causing great pain to patients and poor compliance. When polyoxyethylene castor oil comes into contact with containers, injection syringes, and infusion bags made of polyvinyl chloride plastics, it will leach out the plasticizer diethyl phthalate, causing toxic reactions. The benzyl alcohol contained in commercially available injections may cause damage to newborns and is forbidden to be used for intramuscular injections in children. Commercially available injections have poor stability when diluted, and crystals often precipitate to form fine precipitates that can block the local blood vessels of the patients. The teniposide solution with a concentration of 1 mg/mL should be used up within 4 hours when stored at room temperature and under normal light-off in order to reduce the possibility of precipitation. The diluted injection often has precipitation during long-term instillation, and it is necessary to observe the precipitation status from time to time during the instillation process.

In recent years, pharmaceutical searchers in China and abroad have devoted themselves to researching new drug delivery systems to reduce or replace polyoxyethylene castor oil to improve the efficacy of drugs and reduce toxic side effects, such as liposomes, phospholipid complexes, Tween-containing pharmaceutical compositions, emulsions for systemic administration, microsphere preparations and gel preparations for local administration, and microemulsion preparations for oral administration, etc. However, these new drug delivery systems still have problems such as the toxicity of surfactants (polyoxyethylene castor oil or Tween 80), high cost, and complex preparation processes, etc.

### Summary of the invention

One object of the present invention is to provide a teniposide injection with good dilution stability and low organic solvent irritation.

Another object of the present invention is to provide a method for preparing the teniposide injection.

The objects of the present invention can be achieved through the following technical solutions:
Provided is a teniposide composition, consisting of teniposide and cyclodextrin, and the mass ratio of teniposide to cyclodextrin is 1:5-20; preferably 1:10-15; further preferably 1:12.

The cyclodextrin is preferably selected from one or more of the group consisting of hydroxypropyl-β-cyclodextrin, sulfobutyl ether-β-cyclodextrin, hydroxypropyl-sulfobutyl ether-β-cyclodextrin, mannosyl-β-cyclodextrin and galactosyl- β-cyclodextrin; preferably sulfobutyl-β-cyclodextrin.

Provided is a use of the teniposide composition of the present invention in the preparation of a teniposide injection or powder injection.

A teniposide injection or powder injection, in addition to the teniposide composition of the present invention, also contains a stabilizer and an additive.

The stabilizer is preferably selected from one or more of the group consisting of polyvinylpyrrolidone, polyvinyl alcohol, sodium lauryl sulfonate, sodium lauryl sulfate, poloxamer, and polyethylene glycol. The mass ratio of teniposide to stabilizer is 1:20-100, more preferably 1:35-80, and still more preferably 1:40-50.

The additive is selected from one or more of the group consisting of osmotic pressure regulators, pH value regulators, metal ion complexing agents, and antioxidants; the osmotic pressure regulators are selected from one or more of the group consisting of propylene glycol, glycerol, and mannitol; the pH value regulators are selected from one or more of the group consisting of hydrochloric acid, sulfuric acid, phosphoric acid, citric acid, sorbic acid, lipoic acid, sodium hydroxide, sodium bicarbonate, glycine, and sodium dihydrogen phosphate; the metal ion complexing agents are selected from one or more of the group consisting of ethylenediaminetetraacetic acid, ethylenediaminetetraacetic acid sodium salt, and ethylenediaminetetraacetic acid potassium salt; the antioxidants are selected from one or more of the group consisting of sodium sulfite, sodium bisulfite, sodium metabisulfite, sodium thiosulfate, and vitamin C. The mass ratio of teniposide to additive is 1:1-10, more preferably 1:1-5.

The teniposide injection or powder injection further preferably mainly comprises the following components: teniposide, cyclodextrin, stabilizer, additive and water for injection; wherein the mass ratio of teniposide to cyclodextrin is 1:5-20; the mass ratio of teniposide to stabilizer is 1:20-100; the mass ratio of teniposide to additive is 1:1-10.

The teniposide injection is preferably prepared mainly through the following steps:
1) cyclodextrin, stabilizers and additives are dissolved in water for injection as an aqueous phase, wherein the final concentration of cyclodextrin in the aqueous phase is 5%-35% of the final volume (g/100mL), preferably 15%-35%;
2) teniposide is dissolved in the organic phase, and the final concentration of teniposide in the organic phase is 0.5%-1% (g/100mL);
3) after thoroughly mixing the organic phase obtained in step 2) with the aqueous phase obtained in step 1), the organic solvent is removed, and the volume is fixed with water for injection to obtain the teniposide composition.

The organic solvent is preferably selected from one or more of the group consisting of acetone, methanol, ethanol and chloroform.

The preparation method of teniposide injection of the present invention includes the following steps:
1) cyclodextrin, stabilizers and additives are dissolved in water for injection as the aqueous phase, where the final concentration of cyclodextrin in the aqueous phase is 5%-35% of the final volume (g/100mL), preferably 15%-35%;
2) teniposide is dissolved in an organic phase, teniposide accounts for 0.5%-1% of the organic phase (g/100mL);
3) after thoroughly mixing the organic phase obtained in step 2) with the aqueous phase obtained in step 1), in a mass ratio of teniposide to cyclodextrin 1:1.5-1.8, the organic solvent is removed, and the volume is fixed with water for injection to obtain the teniposide composition.

The organic solvent is preferably selected from one or more of the group consisting of acetone, methanol, ethanol and chloroform.

The step of removing the organic solvent is: the organic phase is added to the aqueous phase while stirring at room temperature, followed by fully stirring for 0.5 hour, further stirring under low negative pressure for 2 hours and stirring under a high vacuum for 1 hour; the low negative pressure condition is -0.01 ∼ -0.03MPa, and the pressure under the high vacuum is -0.1MPa.

### Beneficial effects:

The teniposide composition of the present invention avoids the use of auxiliary materials which have relatively large side effects such as benzyl alcohol, dimethylacetamide and polyoxyethylene castor oil, which can predictably reduce the side effects caused by the auxiliary materials and improve the compliance of patients.

During the dilution process, the commercially available generic preparations need to be operated gently, injecting the injection into the dilution solvent too violently will increase the possibility of precipitation through crystallization. The preparation of the present invention does not have this defect, which reduces the risk of impact of preparation of injection on the quality of the final injection and is convenient for medical staff to use.

The teniposide composition of the present invention successfully solves the disadvantages of the commercially available teniposide injection that it can easily precipitate and form a precipitate during the dilution process, reduces the risk of infusion needle or blood vessel blockage, and is convenient for clinical use.

The invention improves the defect that the product is unstable during the dilution process by optimizing the process of extracting the organic solvent under reduced pressure.

### Specific mode for carrying out embodiments

### Example 1

600mg of sulfobutyl-β-cyclodextrin, 2000mg of PEG300, 60mg of PVP k12, and 60mg of citric acid were dissolved in 3mL of water for injection to prepare an aqueous phase. To 50mg of teniposide were added 2.5mL of ethanol and 2.5mL of acetone to prepare an organic phase.

At room temperature, the organic phase was added to the water phase while stirring, after stirring thoroughly for 0.5 hour, stirring for 2 hours under low negative pressure (-0.01 ∼ -0.03MPa), and stirring for 1 hour under high vacuum (approximately -0.1MPa), the volume was fixed with water for injection to 5mL to obtain a teniposide injection preparation. The obtained teniposide injection preparation was sealed and stored at 4°C, which can be used directly as an intravenous injection, or can be added to a 5% glucose solution or physiological saline for intravenous infusion.

### Example 2

1000mg of sulfobutyl-β-cyclodextrin, 1700mg of PEG400, 60mg of poloxamer, and 60mg of glycine were dissolved in 3mL of water for injection to prepare an aqueous phase. To 50mg of teniposide were added 5mL of ethanol to prepare an organic phase. At room temperature, the organic phase was added to the water phase while stirring, after stirring thoroughly for 0.5 hour, stirring for 2 hours under low negative pressure (-0.01 ∼ -0.03MPa), and stirring for 1 hour under high vacuum (approximately -0.1MPa), the volume was fixed with water for injection to 5mL to obtain a teniposide injection preparation. The obtained teniposide injection preparation was sealed and stored at 4°C, which can be used directly as an intravenous injection, or can be added to a 5% glucose solution or physiological saline for intravenous infusion.

### Example 3

600mg of sulfobutyl-β-cyclodextrin, 2000mg of PEG400, 80mg of PVP k17, and 60mg of lipoic acid were dissolved in 3mL of water for injection to prepare an aqueous phase. To 50mg of teniposide were added 5mL of ethanol to prepare an organic phase. At room temperature, the organic phase was added to the water phase while stirring, after stirring thoroughly for 0.5 hour, stirring for 2 hours under low negative pressure (-0.01 ∼ -0.03MPa), and stirring for 1 hour under high vacuum (approximately -0.1MPa), the volume was fixed with water for injection to 5mL to obtain a teniposide injection preparation. The obtained teniposide injection preparation was sealed and stored at 4°C, which can be used directly as an intravenous injection, or can be added to a 5% glucose solution or physiological saline for intravenous infusion.

### Example 4

800mg of sulfobutyl-β-cyclodextrin, 2000mg of PEG400, 100mg of PVP k30, and 80mg of lipoic acid were dissolved in 3mL of water for injection to prepare an aqueous phase. To 50mg of teniposide were added 5mL of acetone to prepare an organic phase. At room temperature, the organic phase was added to the water phase while stirring, after stirring thoroughly for 0.5 hour, stirring for 2 hours under low negative pressure (-0.01 ∼ -0.03MPa), and stirring for 1 hour under high vacuum (approximately -0.1MPa), the volume was fixed with water for injection to 5mL to obtain a teniposide injection preparation. The obtained teniposide injection preparation was sealed and stored at 4°C, which can be used directly as an intravenous injection, or can be added to a 5% glucose solution or physiological saline for intravenous infusion.

### Example 5

600 mg of sulfobutyl-β-cyclodextrin, 2400 mg of PEG400, 60 mg of PVP k30, and 60 mg of glycine were dissolved in 3 mL of water for injection to prepare an aqueous phase. To 50mg of teniposide were added 5mL of acetone to prepare an organic phase. At room temperature, the organic phase was added to the water phase while stirring, after stirring thoroughly for 0.5 hour, stirring for 2 hours under low negative pressure (-0.01 ∼ -0.03MPa), and stirring for 1 hour under high vacuum (approximately -0.1MPa), the volume was fixed with water for injection to 5mL to obtain a teniposide injection preparation. The obtained teniposide injection preparation was sealed and stored at 4°C, which can be used directly as an intravenous injection, or can be added to a 5% glucose solution or physiological saline for intravenous infusion.

### Example 6

500mg of sulfobutyl-β-cyclodextrin, 2400mg of PEG400, 100mg of PVP k30, and 25mg of glycine were dissolved in 3mL of water for injection to prepare an aqueous phase. To 25mg of teniposide were added 5mL of acetone to prepare an organic phase. The organic phase was added to the water phase while stirring at room temperature. After fully stirring for 1 hour, stirring for 2 hours under low negative pressure (-0.01 ∼ -0.03MPa), and stirring for 1 hour under high vacuum (about -0.1MPa), the volume was fixed with water for injection to 5mL to obtain a teniposide injection preparation. The obtained teniposide injection preparation was sealed and stored at 4°C, which can be used directly as an intravenous injection, or can be added to a 5% glucose solution or physiological saline for intravenous infusion.

### Example 7

500mg of sulfobutyl-β-cyclodextrin, 1800mg of PEG400, 200mg of PVP k30, and 50mg of glycine were dissolved in 3mL of water for injection to prepare an aqueous phase. To 25mg of teniposide were added 5mL of acetone to prepare an organic phase. The organic phase was added to the water phase while stirring at room temperature. After fully stirring for 1 hour, stirring for 2 hours under low negative pressure (-0.01 ∼ -0.03MPa), and stirring for 1 hour under high vacuum (about -0.1MPa), the volume was fixed with water for injection to 5mL to obtain a teniposide injection preparation. The obtained teniposide injection preparation was sealed and stored at 4°C, which can be used directly as an intravenous injection, or can be added to a 5% glucose solution or physiological saline for intravenous infusion.

### Example 8

250mg of sulfobutyl-β-cyclodextrin, 2500mg of PEG400, and 250mg of glycine were dissolved in 3mL of water for injection to prepare an aqueous phase. To 50mg of teniposide were added 5mL of acetone to prepare an organic phase. The organic phase was added to the water phase while stirring at room temperature. After fully stirring for 1 hour, stirring for 2 hours under low negative pressure (-0.01 ∼ -0.03MPa), and stirring for 1 hour under high vacuum (about -0.1MPa), the volume was fixed with water for injection to 5mL to obtain a teniposide injection preparation. The obtained teniposide injection preparation was sealed and stored at 4°C, which can be used directly as an intravenous injection, or can be added to a 5% glucose solution or physiological saline for intravenous infusion.

### Example 9

1000mg of hydroxypropyl-β-cyclodextrin, 1700mg of PEG400, 60mg of poloxamer, and 60mg of glycine were dissolved in 3mL of water for injection to prepare an aqueous phase. To 50mg of teniposide were added 5mL of ethanol to prepare an organic phase. At room temperature, the organic phase was added to the water phase while stirring, after stirring thoroughly for 0.5 hour, stirring for 2 hours under low negative pressure (-0.01 ∼ -0.03MPa), and stirring for 1 hour under high vacuum (approximately -0.1MPa), the volume was fixed with water for injection to 5mL to obtain a teniposide injection preparation. The obtained teniposide injection preparation was sealed and stored at 4°C, which can be used directly as an intravenous injection, or can be added to a 5% glucose solution or physiological saline for intravenous infusion.

### Example 10

1000mg of hydroxypropyl-sulfobutyl-β-cyclodextrin, 1700mg of PEG400, 60mg of poloxamer, and 60mg of glycine were dissolved in 3mL of water for injection to prepare an aqueous phase. To 50mg of teniposide were added 5mL of ethanol to prepare an organic phase. At room temperature, the organic phase was added to the water phase while stirring, after stirring thoroughly for 0.5 hour, stirring for 2 hours under low negative pressure (-0.01 ∼ -0.03MPa), and stirring for 1 hour under high vacuum (approximately -0.1MPa), the volume was fixed with water for injection to 5mL to obtain a teniposide injection preparation. The obtained teniposide injection preparation was sealed and stored at 4°C, which can be used directly as an intravenous injection, or can be added to a 5% glucose solution or physiological saline for intravenous infusion.

### Comparative example 1

Comparative example 1 was carried out according to the commercially available teniposide injection prescription, 50mg of teniposide, 300mg of N,N-dimethylacetamide, 150mg of benzyl alcohol, 2500mg of polyoxyethylene castor oil, appropriate amount of maleic acid which adjusts the pH to about 5 and 42.7% (v/v, based on the total volume of the preparation, the same below) of absolute ethanol, were stirred well to serve as a commercially available control group for comparison in dilution stability.

### Comparative example 2

600mg of sulfobutyl-β-cyclodextrin were dissolved in 5mL of water for injection to prepare an aqueous phase. To 50mg of teniposide were added 5mL of acetone to prepare an organic phase. The organic phase was added to the water phase while stirring at room temperature. After fully stirring for 1 hour, stirring for 2 hours under low negative pressure (-0.01 ∼ -0.03MPa), and stirring for 1 hour under high vacuum (about -0.1MPa), crystallization occurred after standing for 1 day.

### Comparative example 3

250mg of sulfobutyl-β-cyclodextrin were dissolved in 5mL of water for injection to prepare an aqueous phase. 50mg of teniposide were added to the water phase. After fully stirring for 3 hours at room temperature, a clear solution could not be obtained.

### Comparative example 4

600mg of sulfobutyl-β-cyclodextrin were dissolved in 5mL of water for injection to prepare an aqueous phase. 50mg of teniposide were added to the water phase. After fully stirring for 3 hours at room temperature, a clear solution could not be obtained.

### Comparative example 5

600mg of sulfobutyl-β-cyclodextrin and 60mg of glycine were dissolved in 5mL of water for injection to prepare an aqueous phase. To 50mg of teniposide were added 5mL of acetone to prepare an organic phase. At room temperature, the organic phase was added to the water phase while stirring, after stirring thoroughly for 1 hour, stirring for 2 hours under low negative pressure (-0.01 ∼ -0.03MPa), and stirring for 1 hour under high vacuum (about -0.1MPa), crystallization occurred after standing for 1 day.

### Comparative example 6

600mg of sulfobutyl-β-cyclodextrin and 60mg of PVPK17 were dissolved in 5mL of water for injection to prepare an aqueous phase. To 50mg of teniposide were added 5mL of acetone to prepare an organic phase. The organic phase was added to the water phase while stirring at room temperature. After fully stirring for 1 hour, stirring for 2 hours under low negative pressure (-0.01 ∼ -0.03MPa), and stirring for 1 hour under high vacuum (about -0.1MPa), crystallization occurred after standing for 1 day.

### Comparative example 7

600mg of sulfobutyl-β-cyclodextrin and 60mg of citric acid were dissolved in 5mL of water for injection to prepare an aqueous phase. To 50mg of teniposide were added 5mL of acetone to prepare an organic phase. The organic phase was added to the water phase while stirring at room temperature. After fully stirring for 1 hour, stirring for 2 hours under low negative pressure (-0.01 ∼ -0.03MPa), and stirring for 1 hour under high vacuum (about -0.1MPa), crystallization occurred after standing for 1 day.

### Comparative example 8

600mg of sulfobutyl-β-cyclodextrin, 2000mg of PEG300, 60mg of PVP k12, and 60mg of citric acid were dissolved in 3mL of water for injection to prepare an aqueous phase. To 50mg of teniposide were added 2.5mL of ethanol and 2.5mL of acetone to prepare an organic phase. The organic phase was added to the water phase while stirring at room temperature. After fully stirring for 3 hours, and stirring for 1 hour in a high vacuum state (about -0.1MPa), the volume was fixed with water for injection to 5 mL to obtain a teniposide composition. The obtained teniposide mixture was sealed and stored at 4°C, which can be used directly as an intravenous injection, or can be added to a 5% glucose solution or physiological saline for intravenous infusion.

### Effect experiment example 1:

Comparative examples 2, 5, 6, 7, and 8 had the phenomenon of precipitation of the main drug during storage. Comparative examples 3 and 4 cannot prepare ideal aqueous solutions for injection. The teniposide compositions prepared by examples 1-8 and comparative examples 1 and 8 were subjected to a dilution stability experiment. The experiment recorded that the teniposide compositions were diluted with physiological saline or 5% glucose solution to 50mL, 100mL, 250mL and 500mL, 6 samples were prepared for each dilution volume and put at room temperature and natural light for 0.5 hour and 12 hours for precipitation. The number of samples with precipitation was recorded. It can be seen from the results of Table 1 and Table 2 that with the increase of the dilution volume and the standing time, the proportion of precipitation in the commercially available generic preparations increased significantly, and the samples prepared in Examples 1-8 had excellent stability in the dilution experiment. Extracting the organic solvent in two steps of low vacuum and high vacuum are more stable than directly extracting the organic solvent in high vacuum. During the dilution operation, we also found that the operations should be as gentle as possible during the absorption and injection of the commercially available generic preparations, otherwise crystallization will occur quickly, and the injections provided by the present invention did not have this phenomenon. The results in Table 1 and Table 2 also indicated that there was no difference in the dilution stability of the commercially available generic preparations and the preparations of the present invention in physiological saline and 5% glucose diluent.

**Table 1: Precipitation of teniposide compositions diluted with physiological saline**

| | 50mL | | 100mL | | 250ml | | 500ml | |
|---|---|---|---|---|---|---|---|---|
| | 0.5h | 12h | 0.5h | 12h | 0.5h | 12h | 0.5h | 12h |
| Example 1 | 0 sample | 0 sample | 0 sample | 0 sample | 0 sample | 0 sample | 0 sample | 0 sample |
| Example 2 | 0 sample | 0 sample | 0 sample | 0 sample | 0 sample | 0 sample | 0 sample | 0 sample |
| Example 3 | 0 sample | 0 sample | 0 sample | 0 sample | 0 sample | 0 sample | 0 sample | 0 sample |
| Example 4 | 0 sample | 0 sample | 0 sample | 0 sample | 0 sample | 0 sample | 0 sample | 0 sample |
| Example 5 | 0 sample | 0 sample | 0 sample | 0 sample | 0 sample | 0 sample | 0 sample | 0 sample |
| Example 6 | 0 sample | 0 sample | 0 sample | 0 sample | 0 sample | 0 sample | 0 sample | 0 sample |
| Example 7 | 0 sample | 0 sample | 0 sample | 0 sample | 0 sample | 0 sample | 0 sample | 0 sample |
| Example 8 | 0 sample | 0 sample | 0 sample | 0 sample | 0 sample | 0 sample | 0 sample | 1 sample |
| Comparative example 1 | 0 sample | 0 sample | 0 sample | 2 samples | 1 sample | 3 samples | 1 sample | 5 samples |
| Comparative example 8 | 0 sample | 2 samples | 0 sample | 1 sample | 0 sample | 2 samples | 0 sample | 1 sample |

**Table 2: Precipitation of teniposide compositions diluted with 5% glucose**

| | 50mL | | 100mL | | 250ml | | 500ml | |
|---|---|---|---|---|---|---|---|---|
| | 0.5h | 12h | 0.5h | 12h | 0.5h | 12h | 0.5h | 12h |
| Example 1 | 0 sample | 0 sample | 0 sample | 0 sample | 0 sample | 0 sample | 0 sample | 0 sample |
| Example 2 | 0 sample | 0 sample | 0 sample | 0 sample | 0 sample | 0 sample | 0 sample | 0 sample |
| Example 3 | 0 sample | 0 sample | 0 sample | 0 sample | 0 sample | 0 sample | 0 sample | 0 sample |
| Example 4 | 0 sample | 0 sample | 0 sample | 0 sample | 0 sample | 0 sample | 0 sample | 0 sample |
| Example 5 | 0 sample | 0 sample | 0 sample | 0 sample | 0 sample | 0 sample | 0 sample | 0 sample |
| Example 6 | 0 sample | 0 sample | 0 sample | 0 sample | 0 sample | 0 sample | 0 sample | 0 sample |
| Example 7 | 0 sample | 0 sample | 0 sample | 0 sample | 0 sample | 0 sample | 0 sample | 0 sample |
| Example 8 | 0 sample | 0 sample | 0 sample | 0 sample | 0 sample | 1 sample | 0 sample | 1 sample |
| Comparative example 1 | 0 sample | 0 sample | 0 sample | 2 samples | 1 sample | 3 sample | 1 sample | 6 samples |
| Comparative example 8 | 0 sample | 2 samples | 0 sample | 2 samples | 0 sample | 2 samples | 0 sample | 1 sample |

The teniposide compositions of the present invention avoid the use of auxiliary materials with large side effects such as benzyl alcohol, dimethylacetamide and polyoxyethylene castor oil, which can predictably reduce the side effects caused by the auxiliary materials and improve the compliance of patients.

During the dilution process, the commercially available generic preparations need to be operated gently. Injecting the injection into the dilution solvent too vigorously will increase the possibility of precipitation through crystallization. The preparations of the present invention do not have this defect, which reduces the risk of impact of the preparation of the drug solution on the quality of the final infusion and is convenient for medical staff to use. The teniposide compositions of the present invention successfully solve the disadvantages of the commercially available teniposide injection that it is easy to precipitate and form a precipitate during the dilution process, reduces the risk of infusion needle or blood vessel blockage, and is convenient for clinical use.

The invention improves the defect that the product is unstable during the dilution process by optimizing the process of extracting the organic solvent under reduced pressure.

## Claims

1. A teniposide composition consisting of teniposide and cyclodextrin, and the mass ratio of teniposide to cyclodextrin is 1:5-20; preferably 1:10-15; more preferably 1:12.

2. The composition according to claim 1, wherein the cyclodextrin is selected from one or more of the group consisting of hydroxypropyl-β-cyclodextrin, sulfobutyl ether-β-cyclodextrin, hydroxypropyl-sulfobutyl ether-β-cyclodextrin, mannosyl-β-cyclodextrin and galactosyl- β-cyclodextrin; preferably sulfobutyl-β-cyclodextrin.

3. Use of the teniposide composition according to claim 1 or 2 in the preparation of a teniposide injection or powder injection.

4. A teniposide injection or powder injection, which contains a stabilizer and an additive in addition to the teniposide composition according to claim 1 or 2.

5. The teniposide injection or powder injection according to claim 4, wherein the stabilizer is selected from one or more of the group consisting of polyvinylpyrrolidone, polyvinyl alcohol, sodium lauryl sulfonate, sodium lauryl sulfate, poloxamer, and polyethylene glycol; and the mass ratio of teniposide to stabilizer is 1:20-100, more preferably 1:35-80, and still more preferably 1:40-50.

6. The teniposide injection or powder injection according to claim 4, wherein the additive is selected from one or more of the group consisting of osmotic pressure regulators, pH value regulators, metal ion complexing agents, and antioxidants; the osmotic pressure regulators are selected from one or more of the group consisting of propylene glycol, glycerol, and mannitol; the pH value regulators are selected from one or more of the group consisting of hydrochloric acid, sulfuric acid, phosphoric acid, citric acid, sorbic acid, lipoic acid, sodium hydroxide, sodium bicarbonate, glycine, and sodium dihydrogen phosphate; the metal ion complexing agents are selected from one or more of the group consisting of ethylenediaminetetraacetic acid, ethylenediaminetetraacetic acid sodium salt, and ethylenediaminetetraacetic acid potassium salt; the antioxidants are selected from one or more of the group consisting of sodium sulfite, sodium bisulfite, sodium metabisulfite, sodium thiosulfate, and vitamin C; and the mass ratio of teniposide to additive is 1:1-10, more preferably 1:1-5.

7. The teniposide injection or powder injection according to claim 4, wherein the teniposide injection or powder injection mainly comprises the following components: teniposide, cyclodextrin, stabilizer, additive and water for injection; wherein the mass ratio of teniposide to cyclodextrin is 1:5-20; the mass ratio of teniposide to stabilizer is 1:20-100; and the mass ratio of teniposide to additive is 1:1-10.

8. The teniposide injection or powder injection according to claim 4, wherein the teniposide injection is prepared mainly through the following steps:
1) cyclodextrin, stabilizers and additives are dissolved in water for injection as an aqueous phase, wherein the final concentration of cyclodextrin in the aqueous phase is 5%-35%, preferably 15%-35%;
2) teniposide is dissolved in an organic solvent as an organic phase, and the final concentration of teniposide in the organic phase is 0.5%-1%; and the organic solvent is selected from one or more of the group consisting of acetone, methanol, ethanol and chloroform;
3) after thoroughly mixing the organic phase obtained in step 2) with the aqueous phase obtained in step 1), the organic solvent is removed, and the volume is fixed with water for injection to obtain the teniposide composition.

9. A method for preparing the teniposide injection according to claim 4, wherein it includes the following steps:
1) cyclodextrin, stabilizers and additives are dissolved in water for injection as an aqueous phase, wherein the final concentration of cyclodextrin in the aqueous phase is 5%-35%, preferably 15%-35%;
2) teniposide is dissolved in an organic solvent as an organic phase, the final concentration of teniposide in the organic phase is 0.5%-1%; and the organic solvent is selected from one or more of the group consisting of acetone, methanol, ethanol and chloroform;
3) after thoroughly mixing the organic phase obtained in step 2) with the aqueous phase obtained in step 1), in a mass ratio of teniposide to cyclodextrin 1:5-20, the organic solvent is removed, and the volume is fixed with water for injection to obtain the teniposide composition.

10. The method according to claim 9, wherein the step of removing the organic solvent is: the organic phase is added to the aqueous phase while stirring at room temperature, followed by fully stirring for 0.5 hour, further stirring under low negative pressure for 2 hours and stirring under a high vacuum for 1 hour; the low negative pressure condition is -0.01 ∼ -0.03MPa, and the pressure under the high vacuum is -0.1MPa.
